# EUROPEAN PATENT APPLICATION

(11) **EP 4 545 098 A1**
(43) Date of publication of application: **30.04.2025**
(21) Application number: 24206795.7
(22) Date of filing: 15.10.2024
(51) Int. Cl.: A61K 47/54, A61K 47/64, A61K 51/04, A61K 51/08, A61P 35/00

(54) **FIBROBLAST ACTIVATION PROTEIN COMPOUND OR SALT WITH ALBUMIN DUAL-BINDING FUNCTION**

(30) Priority: 26.10.2023 TW 112141037
(71) Applicant: National Atomic Research Institute, Taoyuan 32546 (TW)
(72) Inventor: Chen, Liang-Cheng, Taoyuan (TW); Lo, Sheng-Nan, Taoyuan (TW); Lo, Wei-Lin, Taoyuan (TW); Farn, Shiou-Shiow, Taoyuan (TW)
(74) Representative: Lang, Christian

(57) **Abstract**

A fibroblast activation protein (FAP) compound or salt is provided with albumin dual-binding function. It is a new target FAP molecule, comprising a payload group, a linker group and a FAP binding group. The linker group has an architecture selected from four architectures to connect to the payload group and the FAP binding group to form the FAP compound. The target FAP molecule has the biological activity of binding to albumin in blood and that of binding to FAP protein. The present invention is combined with radioactive nuclide Lu-177, Ac-225, Ga-67 or In-111 for long-circulation FAP targeting in the body. Higher accumulation in expressing FAP tumors is achieved, and the accumulation time of inhibitors in tumors is prolonged. The present invention allows radioactivity to act in the tumor for a long time, thereby reducing the radioactivity concentration or reducing the frequency of radioactive inhibitor administration to inhibit tumor growth.

## Description

### Technical Field of the Invention

The present invention relates to fibroblast activation protein (FAP) compound or salt; more particularly, to a cancer cell-targeting drug which is a FAP inhibitor for long-circulation in the body, where the radioactivity of single dose is reduced and the radioactivity of accumulation in the tumor is increased.

### Description of the Related Arts

At present, most quinoline FAP inhibitors are molecules with short biological circulation. Although it rapidly accumulates in tumors, the accumulation in tumors also decreases with time. If higher radioactivity accumulation is required in the tumor, the injection volume and dosage of inhibitors will also increase. Therefore, it also increases the biological dosimetry in a single administration. However, the better is the lower the biological dosimetry from a single administration. For this reason, in view of the deficiencies in the conventional technology, there is an urgent need for improvement. It is necessary to improve the existing deficiencies and develop a set of FAP inhibitors that is long-retention and long-inhibition in tumors and long-circulation in the body. Hence, the prior arts do not fulfill all users' requests on actual use.

### Summary of the Invention

The main purpose of the present invention is to provide a FAP inhibitor of FAP compound or salt with albumin dual-binding function, where the compound or salt is used for long-circulation in the body, reducing the radioactivity of single dose, and increasing the radioactivity of accumulation in tumors.

In order to achieve the above purpose, the present invention is a FAP compound or salt with albumin dual-binding function, comprising a payload group, a linker group and a FAP (FAP) binding group, where the linker group connects the payload group and the FAP binding group; the payload group is 1,4,7,10-tetraazacyclododecane-1,4,7,10-tetraacetic acid (DOTA) chelator, 1,4,7-triaza Cyclononane-l-glutaric acid-4,7-diacetic acid (DOTAGA) chelator, diethylenetriamine-pentaacetic acid (DTPA) chelator, methotrexate chemotherapy drugs, or active folic acid chemotherapy drug; the linker group is a first architecture, a second architecture, a third architecture, and a fourth architecture; the first architecture comprises a first linker group, a second linker group and an albumin charge interaction group; the second architecture is the second linker group; the third architecture comprises the first linker group and the albumin charge interaction group; the fourth architecture comprises the first linker group and the second linker group; and the FAP binding group is small molecules, peptides, polypeptides, antibodies, proteins, nucleic acids or other biological activity molecules that bind to the FAP protein. Accordingly, a novel FAP compound or salt with albumin dual-binding function is obtained.

In an example of the present invention, when the payload group is the DOTA chelator, DOTAGA chelator or DTPA chelator, it is combined with a radioactive nuclide metal lithium-177 (Lu-177) or actinium-225 (Ac -225). Therefore, the FAP compound or salt with albumin dual-binding function kills cells expressing FAP protein in vitro or in vivo.

In an example of the present invention, when the payload group is the DOTA chelator, DOTAGA chelator or DTPA chelator, it is combined with a radioactive nuclide metal gallium-67 (Ga-67) or indium-111 (In-111). Therefore, the FAP compound or salt with albumin dual-binding function has cells expressing FAP protein in vivo imaged.

In an example of the present invention, when the payload group is the methotrexate chemotherapy drug or the folinic acid chemotherapy drug, the FAP compound or salt with albumin dual-binding function kills cells expressing FAP protein in vitro or in vivo.

In an example of the present invention, in the first architecture of the linker group, the first linker group is connected to the payload group, the second linker group and the albumin charge interaction group; and the second linker group is connected to the FAP binding group.

In an example of the present invention, the first linker group comprises a first structure and a second structure. The first structure is glycine, beta-alanine, γ-aminobutyric acid, 5-aminovaleric acid, or 6-aminohexanoic acid. Each of the first structure, second structure and payload group forms an amide bond, respectively. The second structure is cysteine, homocysteine, 5-mercapto-norvaline, 6-mercaptonorleucine, or 2-amino-7-sulfanylheptanoic acid. Each of the second structure, first structure and second linker group forms an amide bond, respectively. The second structure and albumin charge interaction group form a bond through a thiol-maleimide reaction.

In an example of the present invention, the second linker group has biological activity of albumin binding, which extends the biological half-life of the FAP compound or salt with albumin dual-binding function in vivo. The second linker group comprises a third structure and a fourth structure. The third structure is O-(2-Aminoethyl)-L-serine, 2-amino-4-(2-amino-ethoxy)-butyric acid, or (2S)-2-amino-3-(3-aminopropoxy)propanoic acid. Each of the third structure, fourth structure and second structure of the first linker group forms an amide bond, respectively. The fourth structure is 4-iodobenzenebutanoic acid or 4-(4-methylphenyl)butanoic acid, which forms an amide bond with the FAP binding group.

In an example of the present invention, the albumin charge interaction group has the biological activity of electrical force interaction with the charged albumin, which extends the biological half-life of the FAP compound or salt with albumin dual-binding function in vivo. The albumin charge interaction group comprises a fifth structure modified with 3-maleimidopropionic acid. The fifth structure is polymerized by amide bond with one or several f units, and the f unit is histidine (H), lysine (K) or arginine (R), and one end of the f unit forms an amide bond with maleimide.

In an example of the present invention, in the second architecture of the linker group, the second linker group is connected to the payload group and the FAP binding group. The second linker group has the biological activity of albumin binding and comprises a third structure and a fourth structure. The third structure is O-(2-Aminoethyl)-L-serine, 2-amino-4-(2-amino-ethoxy)-butyric acid, or (2S)-2-amino-3-(3-aminopropoxy)propanoic acid. Each of the third structure, fourth structure and payload group forms an amide bond, respectively. The fourth structure is 4-iodobenzenebutanoic acid or 4-(4-methylphenyl)butanoic acid, which forms an amide bond with the FAP binding group.

In an example of the present invention, in the third architecture of the linker group, the first linker group is connected to the payload group, the albumin charge interaction group and the FAP binding group.

In an example of the present invention, the first linker group comprises a first structure and a second structure. The first structure is glycine, beta-alanine, γ-aminobutyric acid, 5-aminovaleric acid, or 6-aminohexanoic acid. Each of the first structure, second structure and payload group forms an amide bond, respectively. The second structure is cysteine, homocysteine, 5-mercapto-norvaline), 6-mercaptonorleucine, or 2-amino-7-sulfanylheptanoic acid. Each of the second structure, first structure and FAP binding group forms an amide bond, respectively. The second structure and the albumin charge interaction group form a bond through a thiol-maleimide reaction.

In an example of the present invention, the albumin charge interaction group has the biological activity of electrical force interaction with charged albumin, which extends the biological half-life of the FAP compound or salt with albumin dual-binding function in vivo. The albumin charge interaction group comprises the fifth structure modified with 3-maleimidopropionic acid. The fifth structure is polymerized by amide bond with one or several f units, and the f unit is histidine (H), lysine (K) or arginine (R), and one end of the f unit forms an amide bond with maleimide.

In an example of the present invention, in the fourth architecture of the linker group, the first linker group is connected to the payload group and the second linker group, and the second linker group is connected to the FAP binding group.

In an example of the present invention, the first linker group comprises a first structure and a second structure. The first structure is glycine, beta-alanine, γ-aminobutyric acid, 5-aminovaleric acid, or 6-aminohexanoic acid. Each of the first structure, second structure and payload group forms an amide bond, respectively. The second structure is cysteine, homocysteine, 5-mercapto-norvaline), 6-mercaptonorleucine, or 2-amino-7-sulfanylheptanoic acid. Each of the second structure, first structure and second linker group forms an amide bond, respectively.

In an example of the present invention, the second linker group has the biological activity of albumin binding. The second linker group comprises a third structure and a fourth structure. The third structure is O-(2-Aminoethyl)-L-serine, 2-amino-4-(2-amino-ethoxy)-butyric acid, or (2S)-2-amino-3-(3-aminopropoxy)propanoic acid. Each of the third structure, fourth structure and second structure of the first linker group forms an amide bond, respectively. The fourth structure is 4-iodobenzenebutanoic acid or 4-(4-methylphenyl)butanoic acid, which forms an amide bond with the FAP binding group.

### Brief Description of the Drawings

The present invention will be better understood from the following detailed description of the preferred embodiment according to the present invention, taken in conjunction with the accompanying drawings, in which
- FIG.1: is the view showing the conjugation unit according to the present invention;
- FIG.2: is the view showing the first architecture of the linker group;
- FIG.3: is the view showing the second architecture of the linker group;
- FIG.4: is the view showing the third architecture of the linker group;
- FIG.5: is the view showing the fourth architecture of the linker group;
- FIG.6: is the view showing the flow chart of radiolabeling;
- FIG.7-FIG.9: are the views showing the Formula (A)-Formula (M); and
- FIG.10~FIG.30: are the views showing the Formula (1)-Formula (21).

### Description of the Preferred Embodiment

The following description of the preferred embodiment is provided to understand the features and the structures of the present invention.

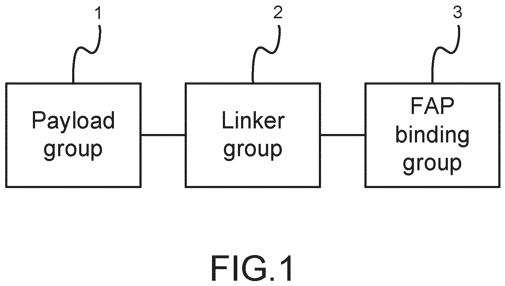

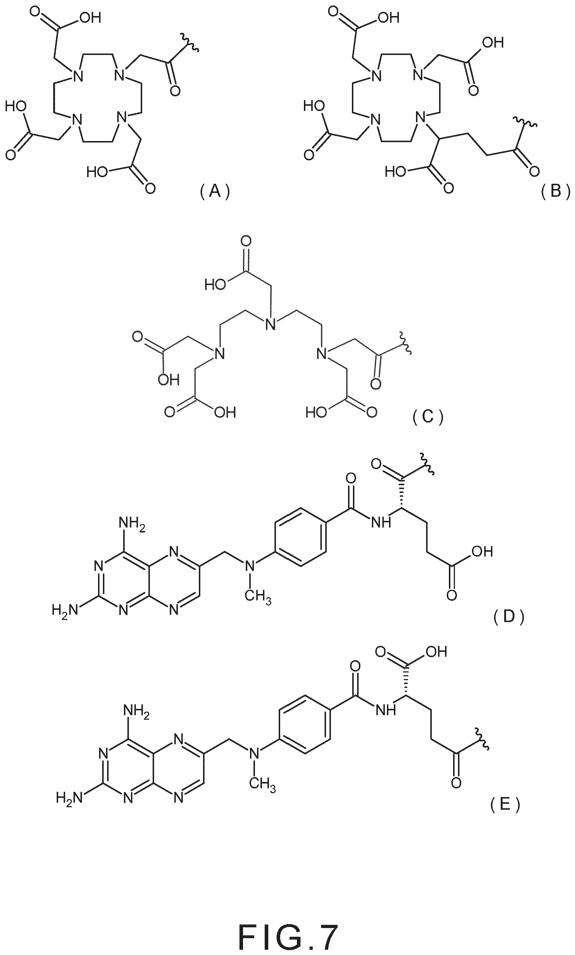

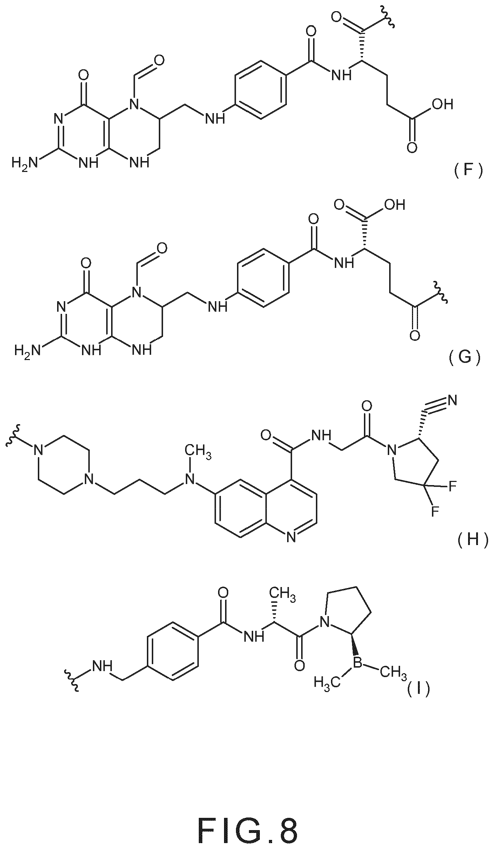

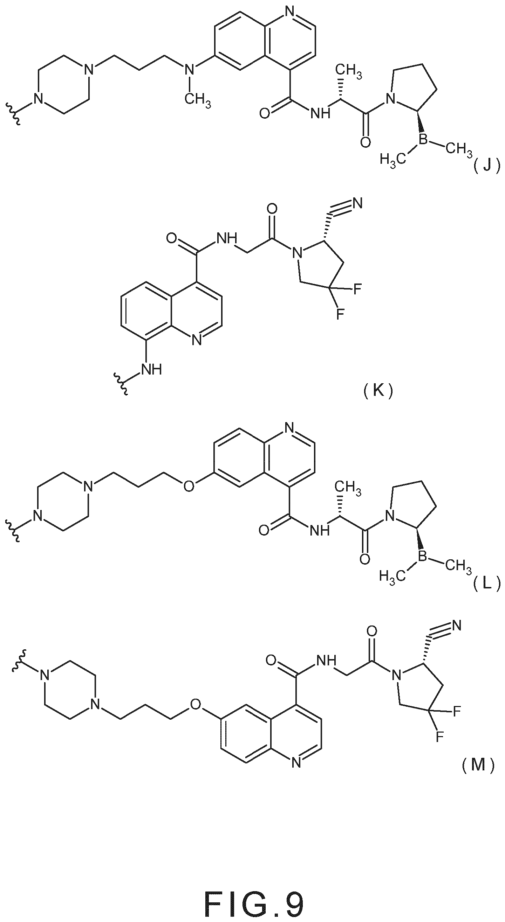

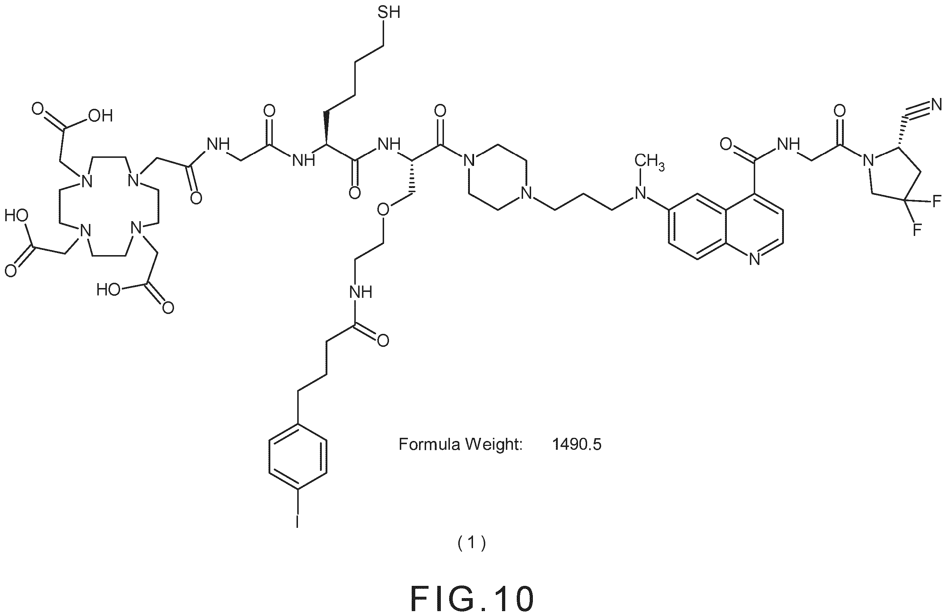

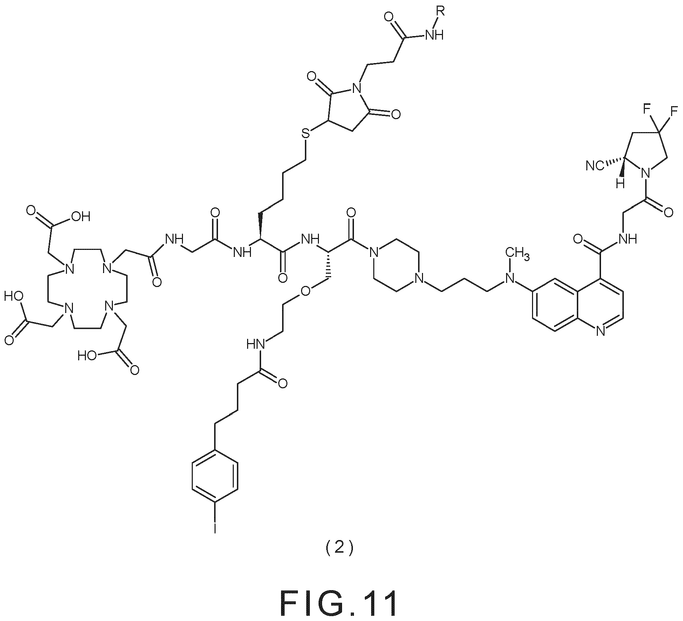

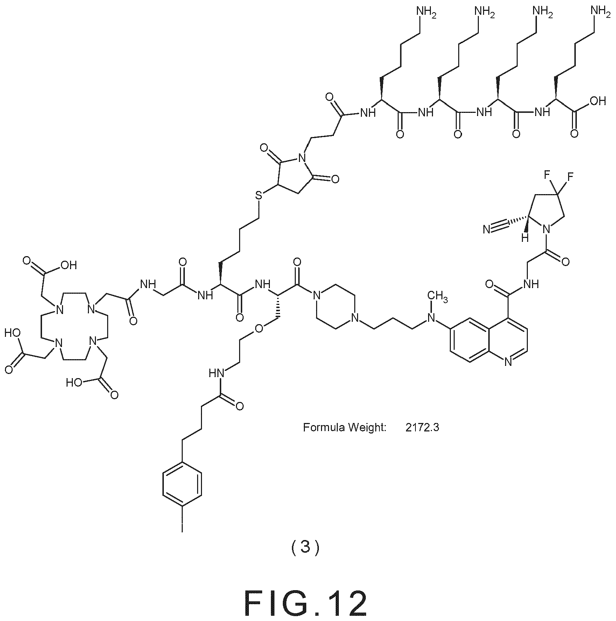

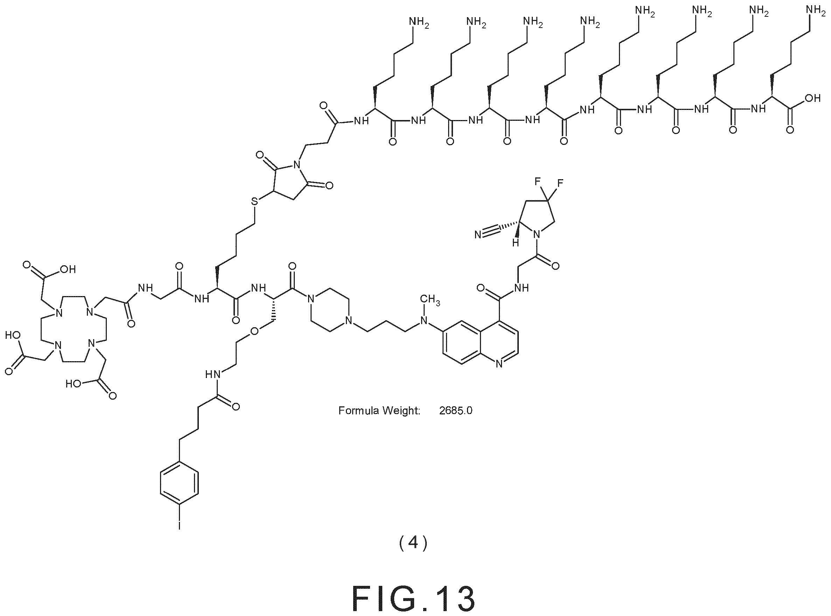

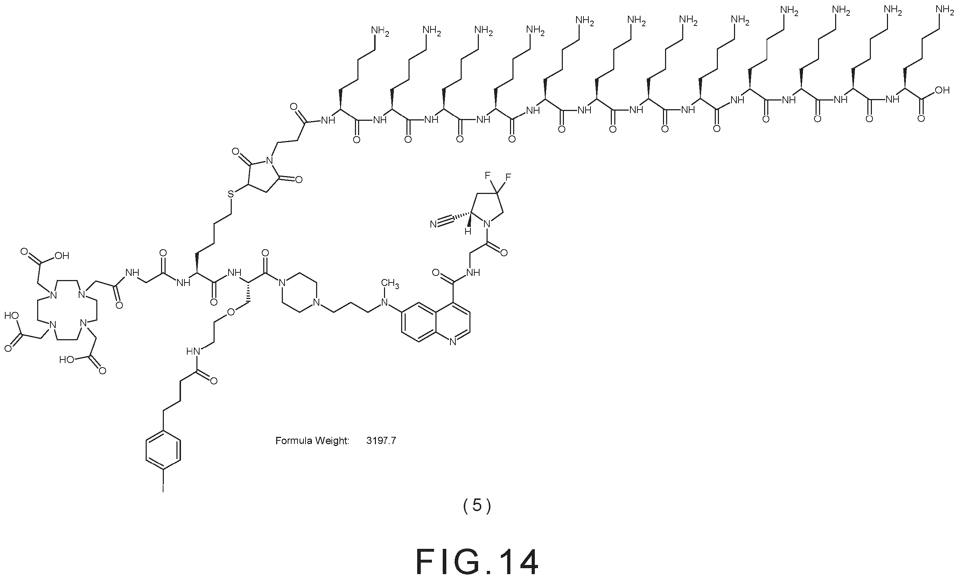

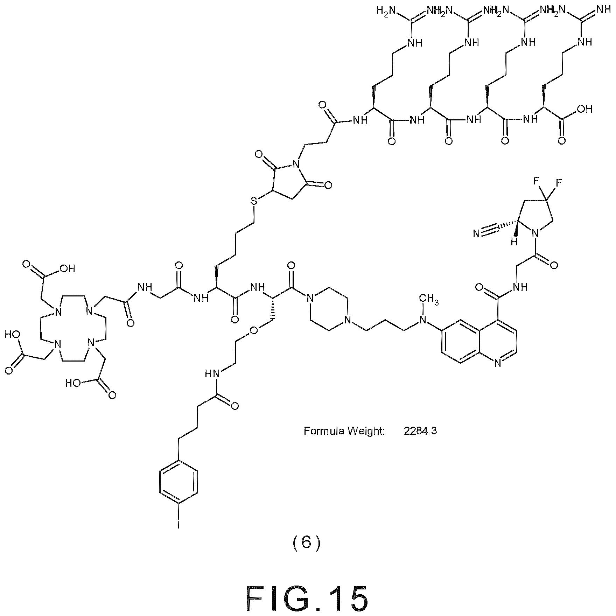

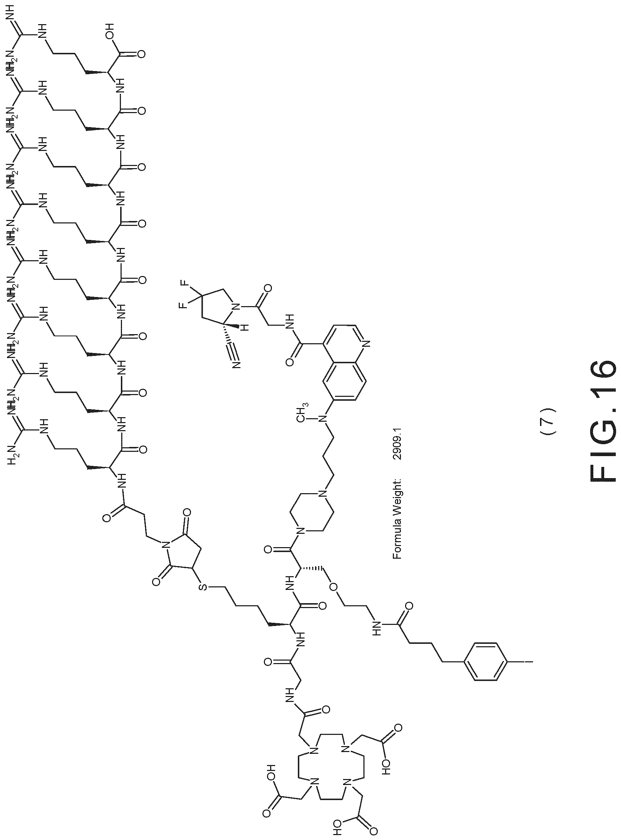

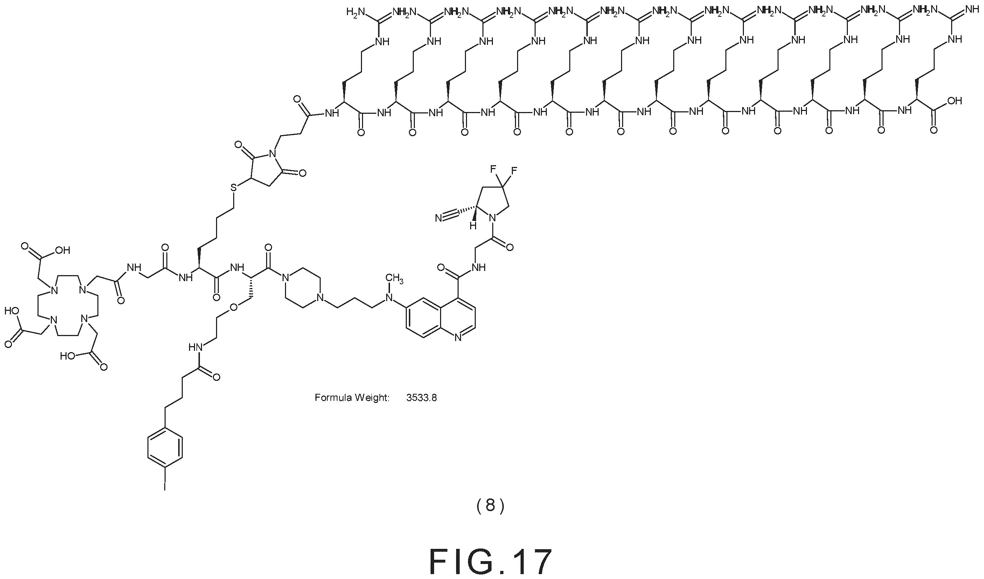

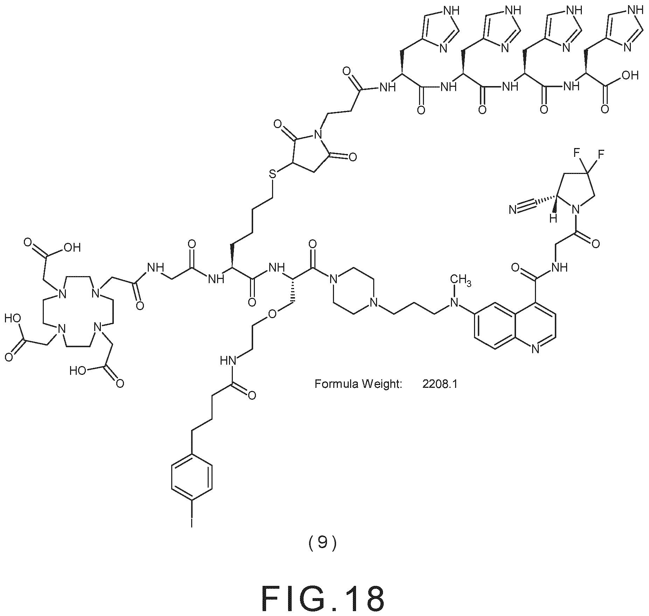

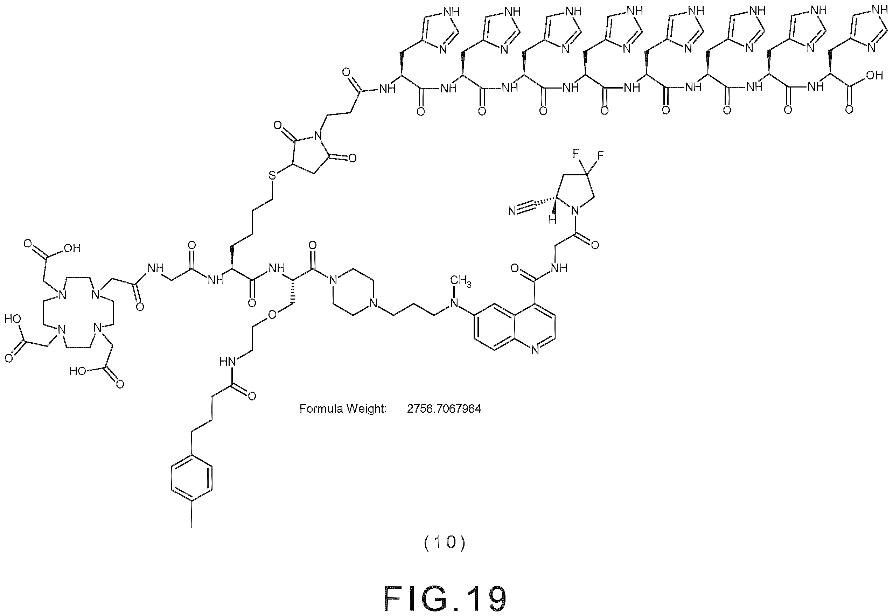

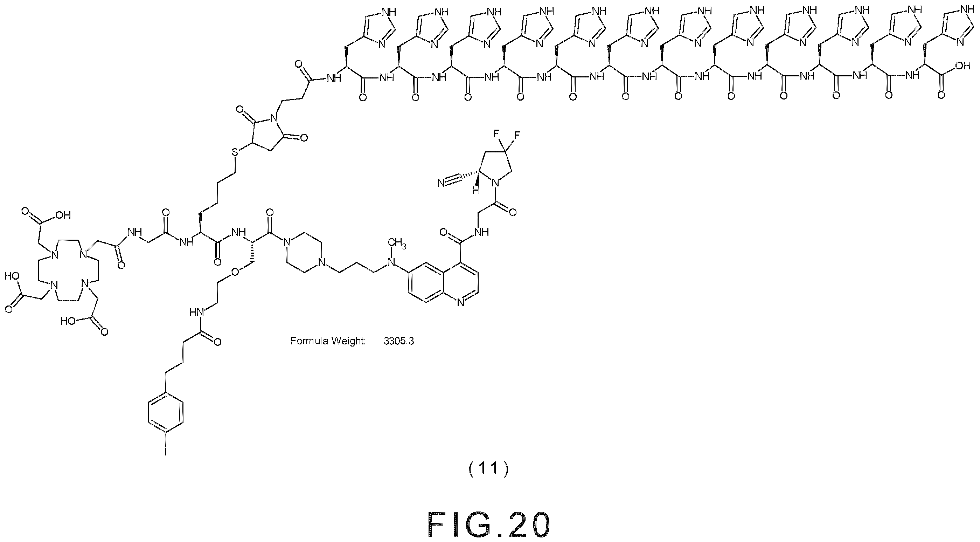

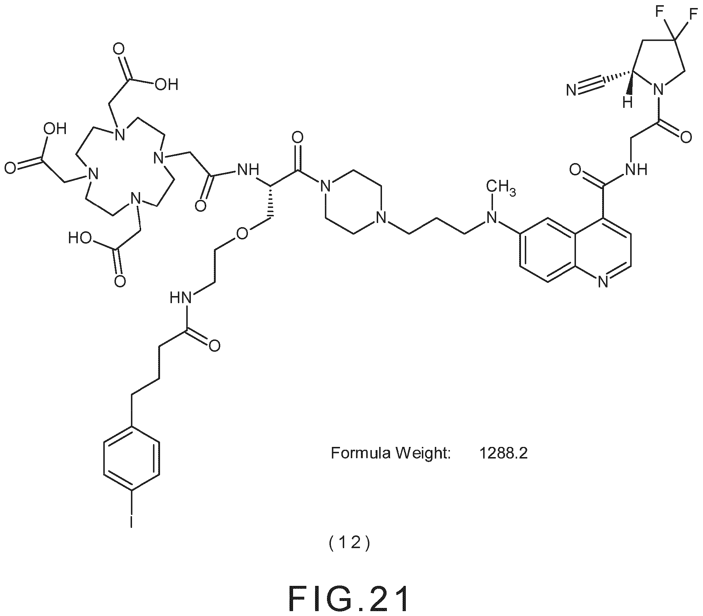

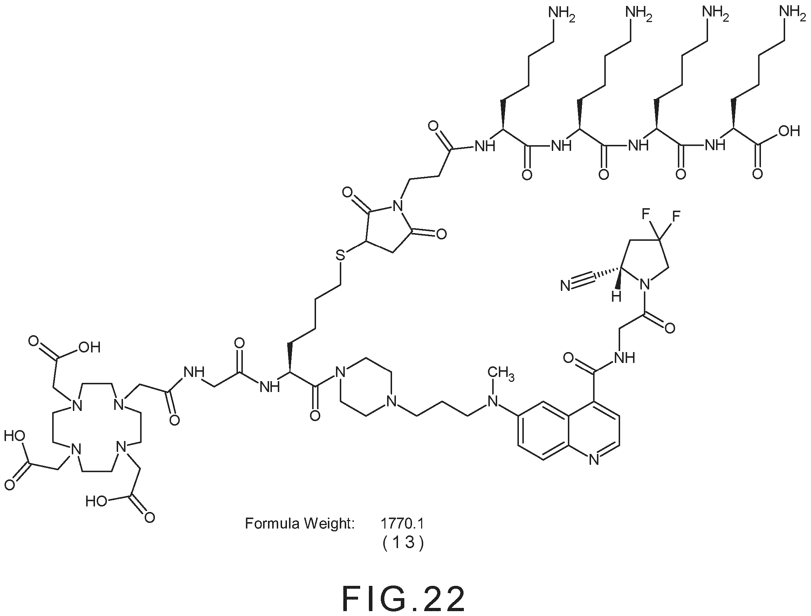

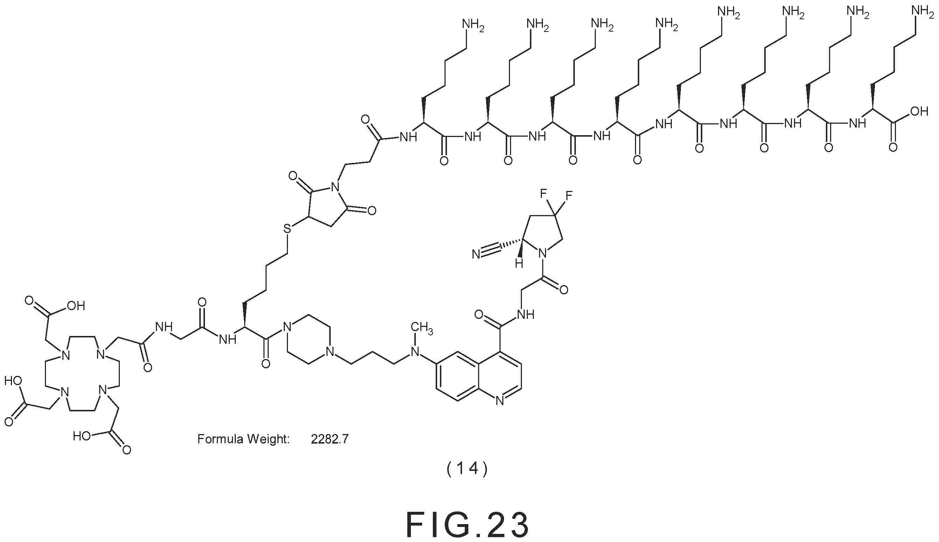

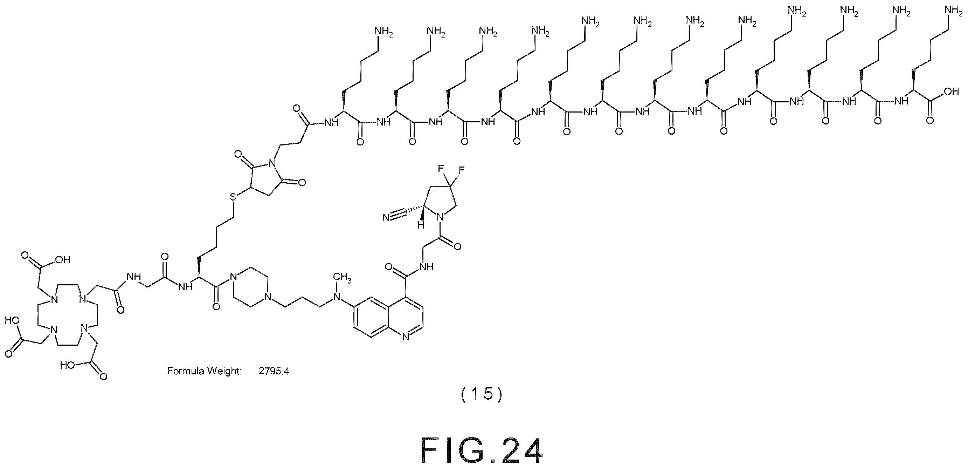

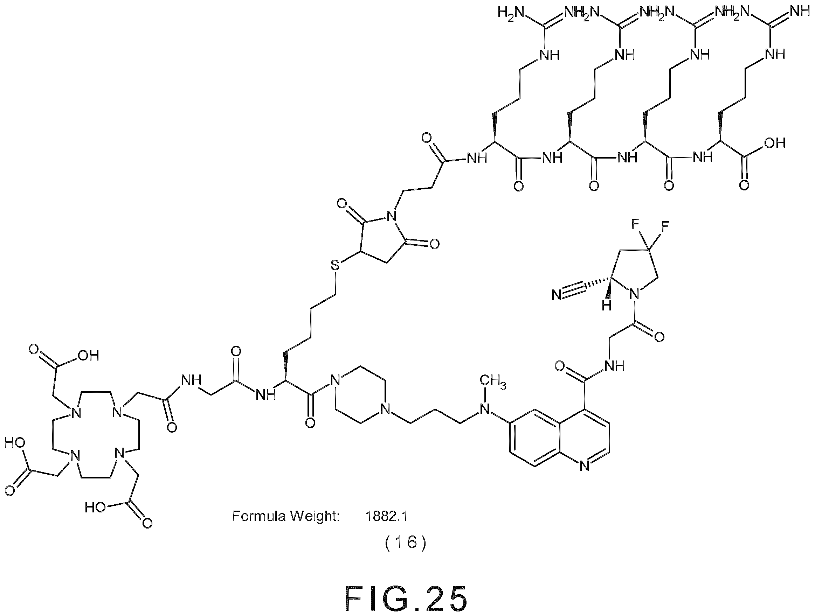

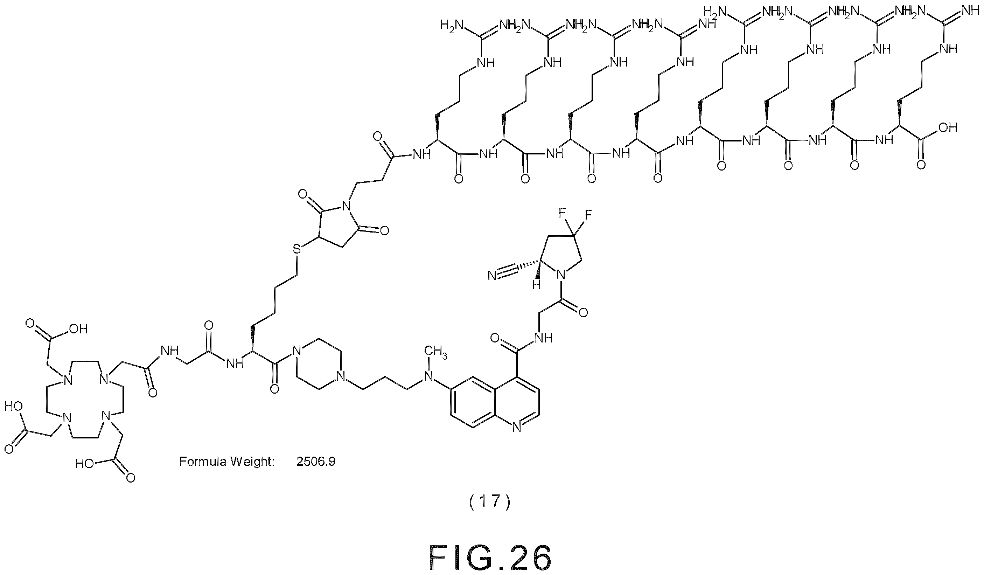

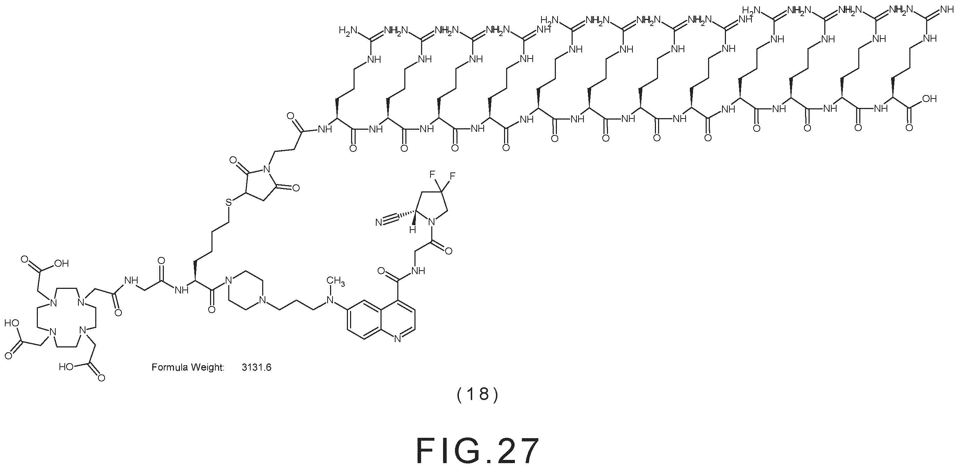

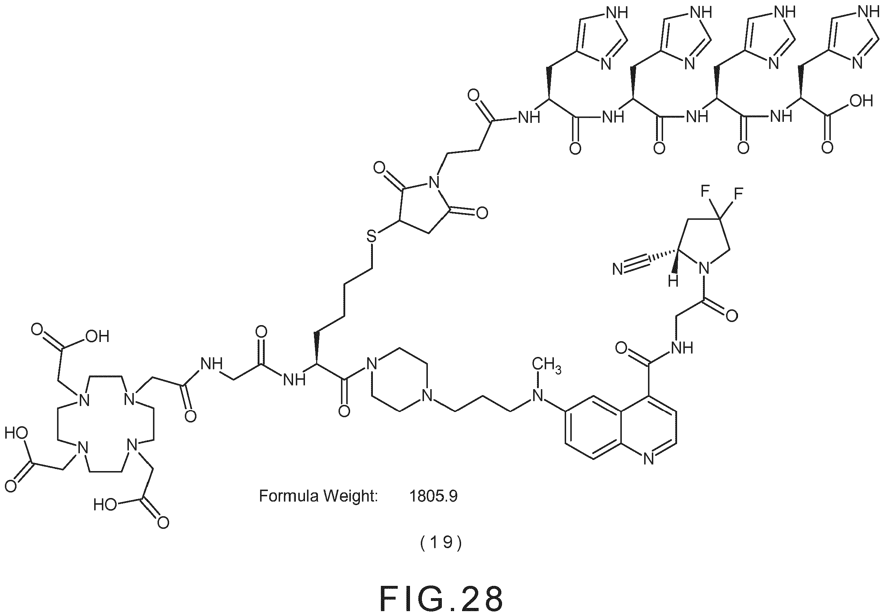

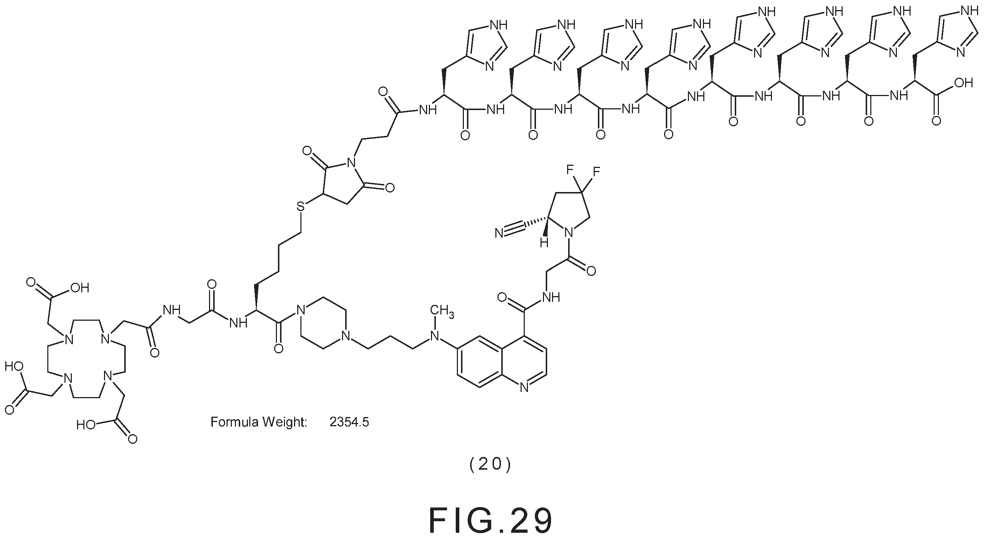

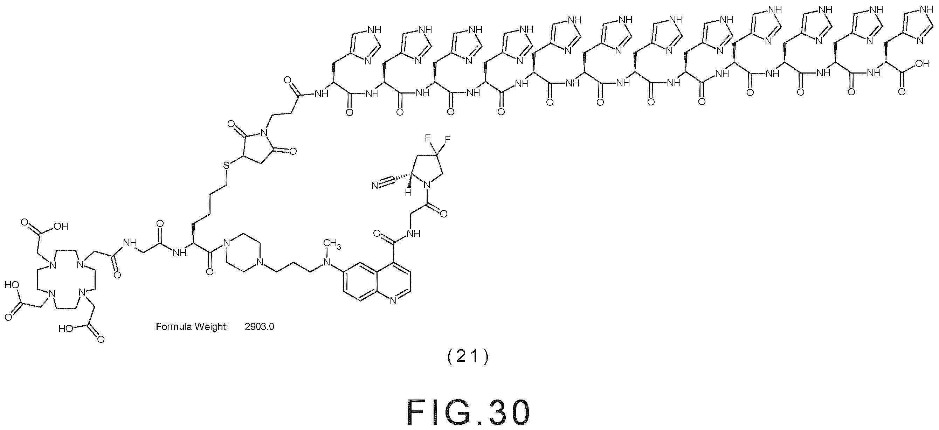

Please refer to FIG.1~FIG.30, which are a view showing a conjugation unit according to the present invention; a view showing a first architecture of a linker group; a view showing a second architecture of the linker group; a view showing a third architecture of the linker group; a view showing a fourth architecture of the linker group; a view showing a flow chart of radiolabeling; views showing Formula (A)-Formula (M); and views showing Formula (1)-Formula (21). As shown in the figures, the present invention is a fibroblast activation protein (FAP) compound or salt with albumin dual-binding function, which comprises a payload group 1, a linker group 2 and a FAP binding group 3 (FIG.1). Therein, the linker group 2 is one of four architectures and is connected to the payload group 1 and the FAP binding group 3 to form the FAP compound or salt with albumin dual-binding function. Therein, the four architectures comprise a first architecture, a second architecture, a third architecture, and a fourth architecture; and the payload group 1 is DOTA (Formula A), DOTAGA (Formula B), DTPA (Formula C), methotrexate (Formula D, Formula E) or folinic acid (Formula F, Formula G).

When the payload group 1 is the DOTA chelator, DOTAGA chelator or DTPA chelator, it is combined with radioactive nuclide metal lithium-177 (Lu-177) or actinium-225 (Ac -225). Therefore, the FAP compound or salt with albumin dual-binding function kills cells expressing FAP protein in vitro or in vivo. When the payload group 1 is the DOTA chelator, DOTAGA chelator or DTPA chelator, it is combined with a radioactive nuclide metal gallium-67 (Ga-67) or indium-111 (In-111). Therefore, the FAP compound or salt with albumin dual-binding function has cells expressing FAP protein in vivo imaged. When the payload group 1 is the methotrexate chemotherapy drug or the folinic acid chemotherapy drug, the FAP compound or salt with albumin dual-binding function kills cells expressing FAP protein in vitro or in vivo.

The FAP binding group 3 has the biological activity for binding to FAP and is peptides, polypeptides, antibodies, proteins, nucleic acids, or small molecules (Formula H to Formula M; broken bond presents the conjugation position).

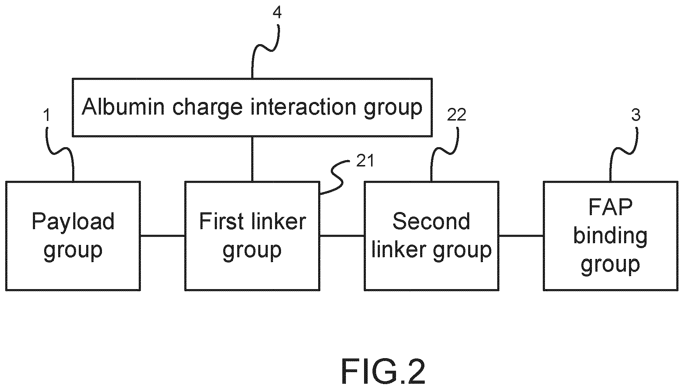

In an example of the present invention (FIG.2), where the first architecture of the linker group 2 includes the albumin charge interaction group 4, the first linker group 21, and the second linker group 22. Therein, the first linker group 21 is connected to the payload group 1, the second linker group 22 and the albumin charge interaction group 4; and the second linker group 22 is connected to the FAP binding group 3.

The first linker group 21 comprises a first structure and a second structure. The first structure is glycine, beta-alanine, γ-aminobutyric acid, 5-aminovaleric acid, or 6-aminohexanoic acid. Each of the first structure, second structure and payload group forms an amide bond, respectively. The second structure is cysteine, homocysteine, 5-mercapto-norvaline, 6-mercaptonorleucine, or 2-amino-7-sulfanylheptanoic acid. Each of the second structure, first structure and second linker group 22 forms an amide bond, respectively. The second structure and the albumin charge interaction group 4 form a bond through a thiol-maleimide reaction.

The second linker group 22 has the biological activity of albumin binding, which extends the biological half-life of the FAP compound or salt with albumin dual-binding function in vivo. The second linker group 22 comprises a third structure and a fourth structure. The third structure is O-(2-Aminoethyl)-L-serine, 2-amino-4-(2-amino-ethoxy)-butyric acid, or (2S)-2-amino-3-(3-aminopropoxy) propanoic acid. Each of the third structure, fourth structure and second structure of the first linker group 21 forms an amide bond, respectively. The fourth structure is 4-iodobenzenebutanoic acid or 4-(4-methylphenyl)butanoic acid, which forms an amide bond with the FAP binding group 3.

The albumin charge interaction group 4 has the biological activity of electrical force interaction with charged albumin, which extends the biological half-life of the FAP compound or salt with albumin dual-binding function in vivo. The albumin charge interaction group 4 comprises a fifth structure modified with 3-maleimidopropionic acid. The fifth structure is polymerized by amide bond with one or several f units, such as f, f-f, f-f-f, f-f-f-f, f-f-f-f-f, f-f-f-f-f-f, f-f-f-f-f-f-f, f-f-f-f-f-f-f-f, f-f-f-f-f-f-f-f-f, f-f-f-f-f-f-f-f-f-f, f-f-f-f-f-f-f-f-f-f-f, f-f-f-f-f-f-f-f-f-f-f-f; the f unit is histidine (H), lysine (K) or arginine (R); and one end of the f unit forms an amide bond with maleimide.

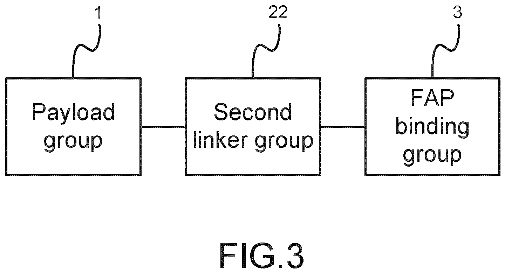

In an example of the present invention (FIG.3), the second architecture of the linker group 2 only has the second linker group 22; and the second linker group 22 is connected to the payload group 1 and the FAP binding group 3.

The second linker group 22 has the biological activity of albumin binding and comprises a third structure and a fourth structure. The third structure is O-(2-Aminoethyl)-L-serine, 2-amino-4-(2-amino-ethoxy)-butyric acid, or (2S)-2-amino-3-(3-aminopropoxy)propanoic acid. Each of the third structure, fourth structure and payload group forms an amide bond, respectively. The fourth structure is 4-iodobenzenebutanoic acid or 4-(4-methylphenyl)butanoic acid, which forms an amide bond with the FAP binding group 3.

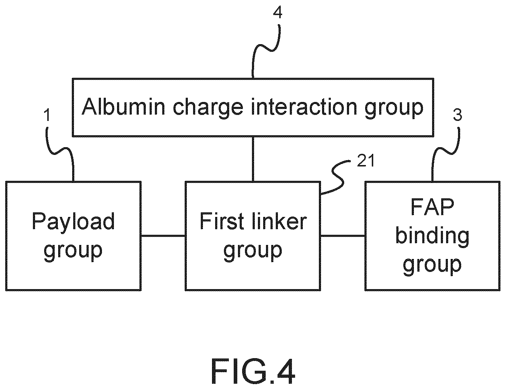

In an example of the present invention (FIG.4), the third architecture of the linker group 2 includes the first linker group 21 and the albumin charge interaction group 4; and the first linker group 21 is connected to the payload group 1, the albumin charge interaction group 4 and the FAP binding group 3.

Therein, the first linker group comprises a first structure and a second structure. The first structure is glycine, beta-alanine, γ-aminobutyric acid, 5-aminovaleric acid, or 6-aminohexanoic acid. Each of the first structure, second structure and payload group forms an amide bond, respectively. The second structure is cysteine, homocysteine, 5-mercapto-norvaline), 6-mercaptonorleucine, or 2-amino-7-sulfanylheptanoic acid. Each of the second structure, first structure and FAP binding group 3 forms an amide bond, respectively. The second structure and the albumin charge interaction group 4 form a bond through a thiol-maleimide reaction.

The albumin charge interaction group 4 has the biological activity of electrical force interaction with charged albumin, which extends the biological half-life of the FAP compound or salt with albumin dual-binding function in vivo. The albumin charge interaction group 4 comprises the fifth structure modified with 3-maleimidopropionic acid. The fifth structure is polymerized by amide bond with one or several f units, such as f, f-f, f-f-f, f-f-f-f, f-f-f-f-f, f-f-f-f-f-f, f-f-f-f-f-f-f, f-f-f-f-f-f-f-f, f-f-f-f-f-f-f-f-f, f-f-f-f-f-f-f-f-f-f, f-f-f-f-f-f-f-f-f-f-f, f-f-f-f-f-f-f-f-f-f-f-f; the f unit is one of histidine (H), lysine (K) or arginine (R); and one end of the f unit forms an amide bond with maleimide.

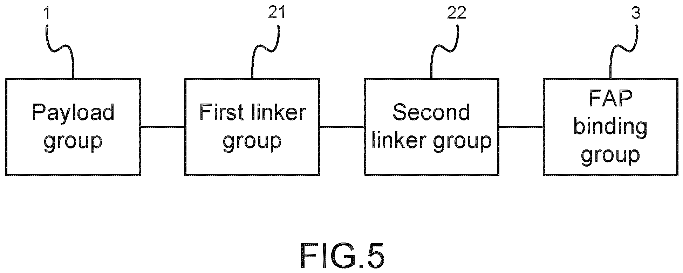

In an example of the present invention (FIG.5), the fourth architecture of the linker group 2 includes the first linker group 21 and the second linker group 22; the first linker group 21 is connected to the payload group 1 and the second linker group 22; and the second linker group 22 is connected to the FAP binding group 3.

The first linker group 21 comprises a first structure and a second structure. The first structure is glycine, beta-alanine, γ-aminobutyric acid, 5-aminovaleric acid, or 6-aminohexanoic acid. Each of the first structure, second structure and payload group forms an amide bond, respectively. The second structure is cysteine, homocysteine, 5-mercapto-norvaline), 6-mercaptonorleucine, or 2-amino-7-sulfanylheptanoic acid. Each of the second structure, first structure and second linker group 22 forms an amide bond, respectively.

The second linker group 22 has the biological activity of albumin binding. The second linker group comprises a third structure and a fourth structure. The third structure is O-(2-Aminoethyl)-L-serine, 2-amino-4-(2-amino-ethoxy)-butyric acid, or (2S)-2-amino-3-(3-aminopropoxy) propanoic acid. Each of the third structure, fourth structure and second structure of the first linker group 21 forms an amide bond, respectively. The fourth structure is 4-iodobenzenebutanoic acid or 4-(4-methylphenyl)butanoic acid, which forms an amide bond with the FAP binding group 3.

The structures of the FAP compound or salt with albumin dual-binding function is represented by the following Formula (1) to Formula (21), but is not limited to the following examples.

Formula (1) presents that the fourth architecture of the linker group 2 (includes the first linker group 21 and the second linker group 22) is connected to the payload group 1 and the FAP binding group 3 to form the FAP compound or salt with albumin dual-binding function.

Formula (2) presents that that the first architecture of the linker group 2 (includes the first linker group 21 and the second linker group 22) is connected to the payload group 1 and the FAP binding group 3 to form the FAP compound or salt with albumin dual-binding function. Therein, R is the albumin charge interaction group 4 selected from - (Lys)4-COOH, -(Lys)8-COOH, -(Lys)12-COOH, -(Arg)4-COOH, -(Arg)8-COOH, -(Arg)12-COOH, -(His)4-COOH, -(His)8-COOH, and -(His)12-COOH.

Formula (3) presents that the first architecture of the linker group 2 (includes the first linker group 21 and the second linker group 22) is connected to the payload group 1 and the FAP binding group 3 to form the FAP compound or salt with albumin dual-binding function. Therein, the albumin charge interaction group 4 is -(Lys)4-COOH.

Formula (4) presents that the first architecture of the linker group 2 (includes the first linker group 21 and the second linker group 22) is connected to the payload group 1 and the FAP binding group 3 to form the FAP compound or salt with albumin dual-binding function. Therein, the albumin charge interaction group 4 is -(Lys)8-COOH.

Formula (5) presents that the first architecture of the linker group 2 (includes the first linker group 21 and the second linker group 22) is connected to the payload group 1 and the FAP binding group 3 to form the FAP compound or salt with albumin dual-binding function. Therein, the albumin charge interaction group 4 is -(Lys)12-COOH.

Formula (6) presents that the first architecture of the linker group 2 (includes the first linker group 21 and the second linker group 22) is connected to the payload group 1 and the FAP binding group 3 to form the FAP compound or salt with albumin dual-binding function. Therein, the albumin charge interaction group 4 is -(Arg)4-COOH.

Formula (7) presents that the first architecture of the linker group 2 (includes the first linker group 21 and the second linker group 22) is connected to the payload group 1 and the FAP binding group 3 to form the FAP compound or salt with albumin dual-binding function. Therein, the albumin charge interaction group 4 is -(Arg)8-COOH.

Formula (8) presents that the first architecture of the linker group 2 (includes the first linker group 21 and the second linker group 22) is connected to the payload group 1 and the FAP binding group 3 to form the FAP compound or salt with albumin dual-binding function. Therein, the albumin charge interaction group 4 is -(Arg)12-COOH.

Formula (9) presents that the first architecture of the linker group 2 (includes the first linker group 21 and the second linker group 22) is connected to the payload group 1 and the FAP binding group 3 to form the FAP compound or salt with albumin dual-binding function. Therein, the albumin charge interaction group 4 is -(His)4-COOH.

Formula (10) presents that the first architecture of the linker group 2 (includes the first linker group 21 and the second linker group 22) is connected to the payload group 1 and the FAP binding group 3 to form the FAP compound or salt with albumin dual-binding function. Therein, the albumin charge interaction group 4 is -(His)8-COOH.

Formula (11) presents that the first architecture of the linker group 2 (includes the first linker group 21 and the second linker group 22) is connected to the payload group 1 and the FAP binding group 3 to form the FAP compound or salt with albumin dual-binding function. Therein, the albumin charge interaction group 4 is -(His)12-COOH.

Formula (12) is presents that the second architecture of the linker group 2 (only includes the second linker group 22) is connected to the payload group 1 and the FAP binding group 3 to form the FAP compound or salt with albumin dual-binding function.

Formula (13) presents that the third architecture of the linker group 2 (only includes the first linker group 21) is connected to the payload group 1 and the FAP binding group 3 to form the FAP compound or salt with albumin dual-binding function. Therein, the albumin charge interaction group 4 is -(Lys)4-COOH.

Formula (14) presents that the third architecture of the linker group 2 (only includes the first linker group 21) is connected to the payload group 1 and the FAP binding group 3 to form the FAP compound or salt with albumin dual-binding function. Therein, the albumin charge interaction group 4 is -(Lys)8-COOH.

Formula (15) presents that the third architecture of the linker group 2 (only includes the first linker group 21) is connected to the payload group 1 and the FAP binding group 3 to form the FAP compound or salt with albumin dual-binding function. Therein, the albumin charge interaction group 4 is -(Lys)12-COOH.

Formula (16) presents that the third architecture of the linker group 2 (only includes the first linker group 21) is connected to the payload group 1 and the FAP binding group 3 to form the FAP compound or salt with albumin dual-binding function. Therein, the albumin charge interaction group 4 is -(Arg)4-COOH.

Formula (17) presents that the third architecture of the linker group 2 (only includes the first linker group 21) is connected to the payload group 1 and the FAP binding group 3 to form the FAP compound or salt with albumin dual-binding function. Therein, the albumin charge interaction group 4 is -(Arg)8-COOH.

Formula (18) presents that the third architecture of the linker group 2 (only includes the first linker group 21) is connected to the payload group 1 and the FAP binding group 3 to form the FAP compound or salt with albumin dual-binding function. Therein, the albumin charge interaction group 4 is -(Arg)12-COOH.

Formula (19) presents that the third architecture of the linker group 2 (only includes the first linker group 21) is connected to the payload group 1 and the FAP binding group 3 to form the FAP compound or salt with albumin dual-binding function. Therein, the albumin charge interaction group 4 is -(His)4-COOH.

Formula (20) presents that the third architecture of the linker group 2 (only includes the first linker group 21) is connected to the payload group 1 and the FAP binding group 3 to form the FAP compound or salt with albumin dual-binding function. Therein, the albumin charge interaction group 4 is -(His)8-COOH.

Formula (21) presents that the third architecture of the linker group 2 (only includes the first linker group 21) is connected to the payload group 1 and the FAP binding group 3 to form the FAP compound or salt with albumin dual-binding function. Therein, the albumin charge interaction group 4 is -(His)12-COOH.

The following examples are only examples for understanding the details and contents of the present invention, but are not used to limit the patentable scope of the present invention.

### [Application Method] Radiolabeling Fibroblast Activation Protein Compound or Salt with Albumin Dual-Binding Function

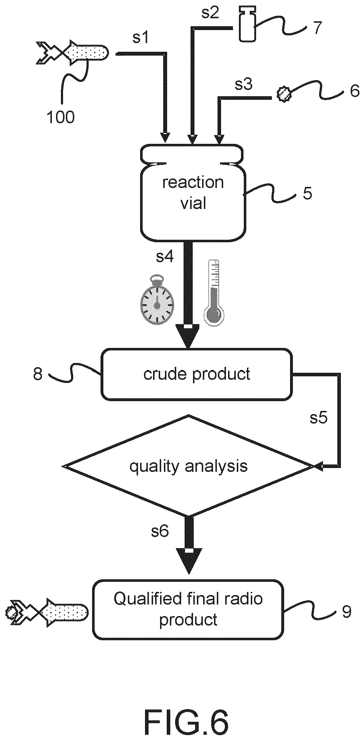

Referring to FIG.6, the FAP compound or salt according to the present invention is presented as INER-FAPI-X 100. For step s1 to s3, the INER-FAPI-X 100, radionuclide metal 6 and radiolabeling buffer 7 are mixed in a reaction vial 5 with the step s4 temperature and incubated time used to yield crude product 8. Then the quality analysis is performed with radio-TLC and radio-HPLC for step s5. The criteria of final radio product 9 for radiochemical purity is over 90% for passing and then the qualified product is used for tumor-bearing mice imaging to evaluate the efficacy of tumor accumulation and expend biological half-life.

### [Preferred Embodiment] Application of radiolabeling INER-FAPI-X in expressing FAP HEK-293 human embryonic kidney cancer tumor model

The HEK-293 human embryonic kidney cancer cells expressing FAP (HEK-293-FAP cells) are inoculated with 4 × 10⁶ cells at the forward right flanks of BALB/c nude mice. The INER-FAPI-X drugs are labeling with the radioactive isotope Lu-177, and the radiochemical purity of Lu-177-INER-FAPI-X or Lu-177-FAPI-46 is greater than 95%. These drugs are prepared to about 100 µL volume in normal saline. The nano-SPECT/CT imaging is performed at 1, 4, and 24 h after the intravenously administrated via tail vein.

The nanoSPECT/CT imaging is performed at 1, 4, and 24 h after administration to monitor the distribution of radiopharmaceuticals in the animal. The images are semi-quantification by using imaging analysis software. The SPECT count value of reference source is obtained by using imaging analysis software. The conversion formula between SPECT count value and actual activity is obtained from the reference source with known activity at each time point. The SPECT count value is obtained from region of image of each target organ, and obtained the activity/volume of each target organ by using the conversion formula. According to the activity and volume of the administered injection, the biodistribution value (%ID/mL) of Lu-177-INER-FAPI-X or Lu-177-FAPI-46 per mL in the blood of heart and tumor is obtained, as shown in Table 1.

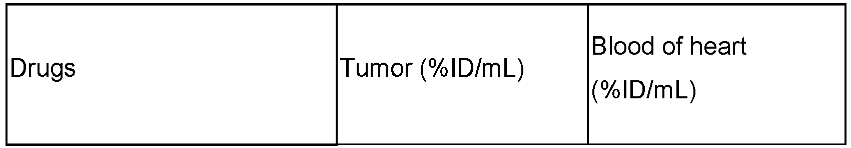

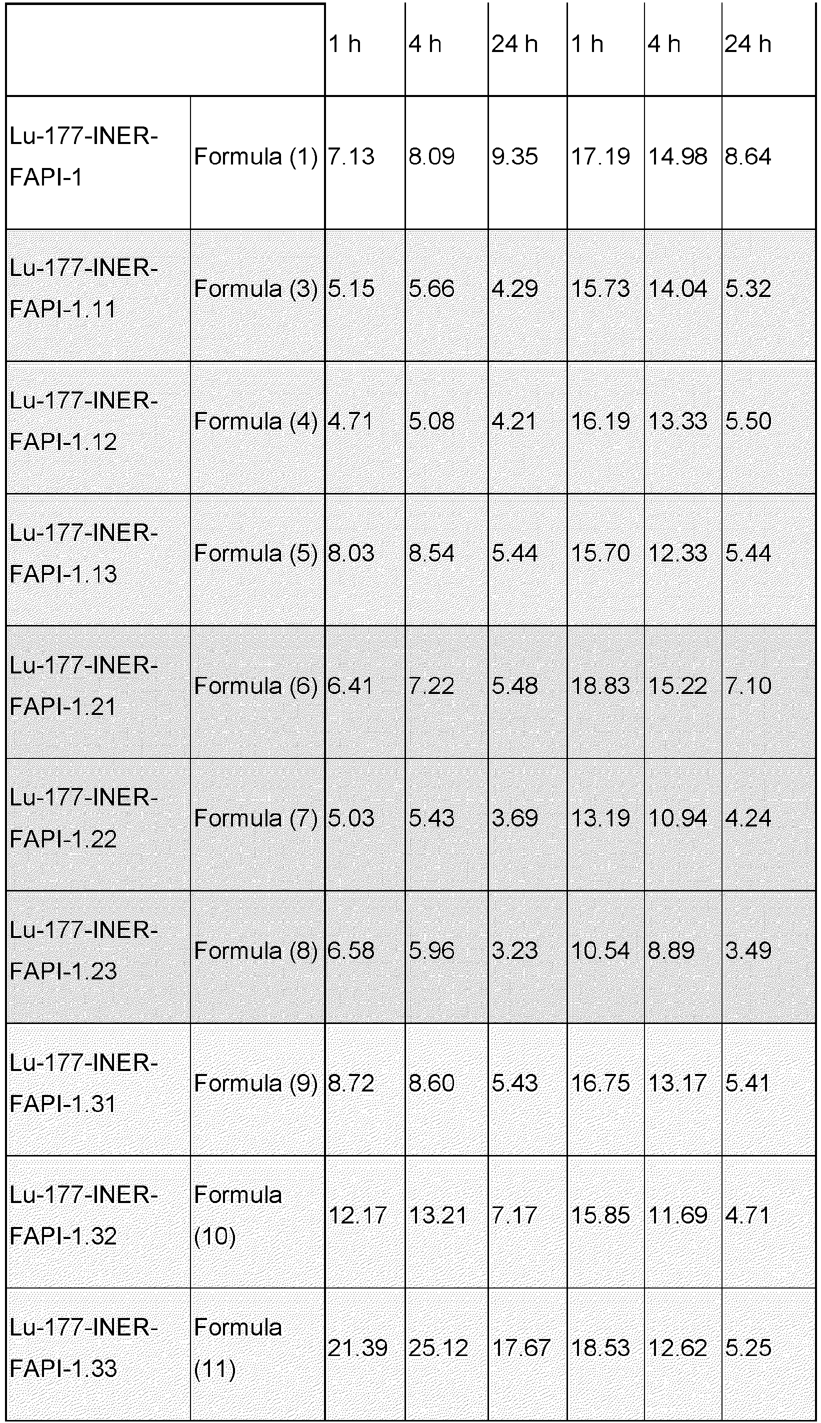

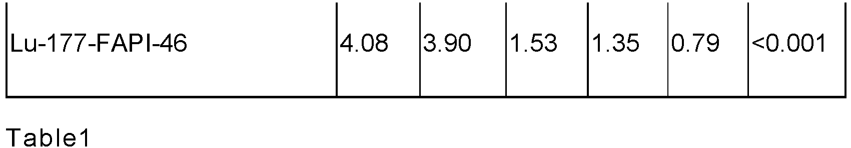

From the quantitative values of animal images above, it is found that the accumulation values of Lu-177-INER-FAPI-X in the tumor are higher than those of Lu-177-FAPI-46 at 1, 4 and 24 h after administration, and the distribution time is prolonged to 24 h. The blood values of heart are significantly higher than those of Lu-177-FAPI-46, indicating that the circulation time in the blood can be increased.

Therefore, the FAP compound or salt with albumin dual-binding function in the present invention is a new target FAP molecule. It has the biological activity of binding to albumin in blood and the biological activity of binding to FAP protein. It is combined with radioactive nuclides Lu-177, Ac-225, Ga-67 or In-111 and used for long-circulation FAP targeting in the body. Compared with the previous radioactive labeled FAP inhibitors, it has higher accumulation in expressing FAP tumors, and prolong the accumulation time of inhibitors in tumors. This allows radioactivity to act in the tumor for a long time, thereby reducing the radioactivity concentration or reducing the frequency of radioactive inhibitor administration to inhibit tumor growth. Therefore, the problem of insufficient accumulation of existing FAP inhibitor drugs in tumors is improved.

To sum up, the present invention is a FAP compound or salt with albumin dual-binding function, where various shortcomings of conventional methods are effectively improved; long-circulation in blood and better tumor cell inhibition effect are achieved; and the accumulation of drug radioactivity in the tumor site is increased to obtain better therapeutic effects.

The preferred embodiment herein disclosed is not intended to unnecessarily limit the scope of the invention. Therefore, simple modifications or variations belonging to the equivalent of the scope of the claims and the instructions disclosed herein for a patent are all within the scope of the present invention.

## Claims

1. A fibroblast activation protein (FAP) compound with albumin dual-binding function,
comprising a payload group, a linker group and a FAP binding group,
said linker group being connected with said payload group and said FAP binding group,
wherein said payload group is selected from a group consisting of 1,4,7,10-tetraazacyclo dodecane-1,4,7,10-tetraacetic acid (DOTA) chelator, 1,4,7-triaza cyclononane-l-glutaric acid-4,7-diacetic acid (DOTAGA) chelator, diethylenetriaminepentaacetic acid (DTPA) chelator, methotrexate chemotherapy drugs, and active folic acid chemotherapy drug;
wherein said linker group has an architecture selected from a group consisting of a first architecture, a second architecture, a third architecture, and a fourth architecture; said first architecture comprises a first linker group, a second linker group and an albumin charge interaction group; said second architecture is said second linker group; said third architecture comprises said first linker group and said albumin charge interaction group; and said fourth architecture comprises said first linker group and said second linker group; and
wherein said FAP binding group is biological activity molecules that bind to the FAP protein and selected from a group consisting of small molecules, peptides, polypeptides, antibodies, proteins, and nucleic acids.

2. The FAP compound according to claim 1,
wherein said payload group is selected from a group consisting of said DOTA chelator, said DOTAGA chelator and said DTPA chelator; said payload group is combined with a radioactive nuclide metal selected from a group consisting of lithium-177 (Lu-177) and actinium-225 (Ac-225); and the FAP compound thus kills cells expressing FAP protein in vitro/in vivo.

3. The FAP compound according to claim 1,
wherein said payload group is selected from a group consisting of said DOTA chelator, said DOTAGA chelator and said DTPA chelator; said payload group is combined with a radioactive nuclide metal selected from a group consisting of gallium-67 (Ga-67) and indium-111 (In-111); and the FAP compound thus images cells expressing FAP protein in vivo.

4. The FAP compound according to claim 1,
wherein said payload group is selected from a group consisting of said methotrexate chemotherapy drug and said active folinic acid chemotherapy drug; and the FAP compound thus kills cells expressing FAP protein in vitro/in vivo.

5. The FAP compound according to claim 1,
wherein, in said first architecture of said linker group, said first linker group is connected to said payload group, said second linker group and said albumin charge interaction group; and said second linker group is connected to said FAP binding group.

6. The FAP compound according to claim 5,
wherein said first linker group comprises a first structure and a second structure; said first structure is selected from a group consisting of glycine, beta-alanine, γ-aminobutyric acid, 5-aminovaleric acid, and 6-aminohexanoic acid; each of said first structure, said second structure and said payload group forms an amide bond, respectively; said second structure is selected from a group consisting of cysteine, homocysteine, 5-mercapto- norvaline), 6-mercaptonorleucine, and 2-amino-7-sulfanylheptanoic acid; each of said second structure, said first structure and said second linker group forms an amide bond, respectively; and said second structure and said albumin charge interaction group form a bond through a thiol-maleimide reaction.

7. The FAP compound according to claim 5,
wherein said second linker group has the biological activity of albumin binding, which extends the biological half-life of the FAP compound in vivo; said second linker group comprises a third structure and a fourth structure; said third structure is selected from a group consisting of O-(2-Aminoethyl)-L-serine, 2-amino-4-(2-amino-ethoxy)-butyric acid, and (2S)-2-amino-3-(3-aminopropoxy)propanoic acid; each of said third structure, said fourth structure and said second structure of said first linker group forms an amide bond, respectively; and said fourth structure is 4-iodobenzenebutanoic acid or 4-(4-methylphenyl)butanoic acid, which forms an amide bond with said FAP binding group.

8. The FAP compound according to claim 5,
wherein said albumin charge interaction group has the biological activity of electrical force interaction with charged albumin, which extends the biological half-life of the FAP compound in vivo; said albumin charge interaction group comprises a fifth structure modified with 3-maleimidopropionic acid; said fifth structure is polymerized by amide bond with at least one f unit, and said f unit is selected from a group consisting of histidine (H), lysine (K) and arginine (R); and one end of said f unit forms an amide bond with maleimide.

9. The FAP compound according to claim 1,
wherein, in said second architecture of said linker group, said second linker group is connected to said payload group and said FAP binding group; said second linker group has the biological activity of albumin binding and comprises said third structure and said fourth structure; said third structure is selected from a group consisting of O-(2-Aminoethyl)-L-serine, 2-amino-4-(2-amino-ethoxy)-butyric acid, and (2S)-2-amino-3-(3-aminopropoxy)propanoic acid; each of said third structure, said fourth structure and said payload group form an amide bond, respectively; and said fourth structure is 4-iodobenzenebutanoic acid or 4-(4-methylphenyl)butanoic acid, which forms an amide bond with said FAP binding group.

10. The FAP compound according to claim 1,
wherein, in said third architecture of said linker group, said first linker group is connected to said payload group, said albumin charge interaction group and said FAP binding group.

11. The FAP compound according to claim 10,
wherein said first linker group comprises said first structure and said second structure; said first structure is selected from a group consisting of glycine, beta-alanine, γ-aminobutyric acid, 5-aminovaleric acid, and 6-aminohexanoic acid; each of said first structure, said second structure and said payload group forms an amide bond, respectively; said second structure is selected from a group consisting of cysteine, homocysteine, 5-mercapto-norvaline, 6-mercaptonorleucine, and 2-amino-7-sulfanylheptanoic acid; each of said second structure, said first structure and said FAP binding group forms an amide bond, respectively; and said second structure and said albumin charge interaction group form a bond through a thiol-maleimide reaction.

12. The FAP compound according to claim 10,
wherein said albumin charge interaction group has the biological activity of electrical force interaction with said charged albumin, which extends the biological half-life of the FAP compound in vivo; said albumin charge interaction group comprises a fifth structure modified with 3-maleimidopropionic acid; said fifth structure is polymerized by amide bond with at least one f units; said f unit is selected from a group consisting of histidine (H), lysine (K) and arginine (R); and one end of said f unit forms an amide bond with maleimide.

13. The FAP compound according to claim 1,
wherein, in said fourth architecture of said linker group, said first linker group is connected to said payload group and said second linker group; and said second linker group is connected to said FAP binding group.

14. The FAP compound according to claim 13,
wherein said first linker group comprises a first structure and a second structure; said first structure is selected from a group consisting of glycine, beta-alanine, γ-aminobutyric acid, 5-aminovaleric acid, and 6-aminohexanoic acid; each of said first structure, said second structure and said payload group forms an amide bond, respectively; said second structure is selected from a group consisting of cysteine, homocysteine, 5-mercapto- norvaline), 6-mercaptonorleucine, and 2-amino-7-sulfanylheptanoic acid; and each of said second structure, said first structure and said second linker group forms an amide bond, respectively.

15. The FAP compound according to claim 13,
wherein said second linker group has the biological activity of albumin binding; said second linker group comprises a third structure and said fourth structure; said third structure is selected from a group consisting of O-(2-Aminoethyl)-L-serine, 2-amino-4-(2-amino-ethoxy)-butyric acid, and (2S)-2-amino-3-(3-aminopropoxy)propanoic acid; each of said third structure, said fourth structure and said second structure of said first linker group forms an amide bond, respectively; and said fourth structure is 4-iodobenzenebutanoic acid or 4-(4-methylphenyl)butanoic acid, which forms an amide bond with said FAP binding group.
